# EUROPÄISCHE PATENTANMELDUNG

(11) **EP 2 607 896 A1**
(43) Veröffentlichungstag der Anmeldung: **26.06.2013**
(21) Anmeldenummer: 12003780.9
(22) Anmeldetag: 14.05.2012
(51) Int. Cl.: G01N 33/02

(54) **Verfahren zur Bestimmung eines Kennwerts für die Frische und/oder den Frischeverlust einer Lebensmittel-Probe**

(30) Priorität: 23.12.2011 DE 102011122234
(71) Anmelder: QFood GmbH, 79194 Gundelfingen (DE)
(72) Erfinder: Seidel, Robert, Dr., 79102 Freiburg (DE); Klapproth, Holger, Dr., 79108 Freiburg (DE); Bednar, Sonja, 79194 Gundelfingen (DE)
(74) Vertreter: Huwer, Andreas

(57) **Zusammenfassung**

Bei einem Verfahren zur Bestimmung eines Kennwerts für die Frische und/oder den Frischeverlust einer zumindest ein erstes Ausgangsgangsprodukt und ein zweites Ausgangsgangsprodukt enthaltenden Lebensmittel-Probe wird beim Auftreten einer ersten Frischeverlustart das erste Ausgangsprodukt zersetzt und es wird mindestens ein erster Metabolit gebildet. Beim Auftreten einer zweiten Frischeverlustart wird das zweite Ausgangsprodukt zersetzt und es wird mindestens ein zweiter Metabolit gebildet. Für die Konzentration des ersten Ausgangsprodukts wird ein erster Ausgangsprodukt-Konzentrationswert und für die Konzentration des ersten Metaboliten ein erster Metabolit-Konzentrationswert gemessen. Für die Konzentration des zweiten Ausgangsprodukts wird ein zweiter Ausgangsprodukt-Konzentrationswert und für die Konzentration des zweiten Metaboliten ein zweiter Metabolit-Konzentrationswert gemessen. Die Ausgangsprodukt-Konzentrationswerte und die Metabolit-Konzentrationswerte werden gleichzeitig gemessen. Der Kennwert für die Frische der Lebensmittelprobe wird in Abhängigkeit vom Verhältnis des ersten Ausgangsprodukt-Konzentrationswerts zum ersten Metabolit-Konzentrationswert und vom Verhältnis des zweiten Ausgangsprodukt-Konzentrationswerts zum zweiten Metabolit-Konzentrationswert ermittelt.

## Beschreibung

Die Erfindung betrifft ein Verfahren zur Bestimmung eines Kennwerts für die Frische und/oder den Frischeverlust einer zumindest ein erstes Ausgangsgangsprodukt und ein zweites Ausgangsgangsprodukt enthaltenden Lebensmittel-Probe.

Inhaltsstoffe von Lebensmitteln wie Vitamine, Nukleinsäuren, Eiweiße und Aminosäuren u. a. unterliegen einer natürlichen Alterung wie beispielsweise Oxidation oder enzymatische Umsetzung als Funktion der Zeit, abhängig z.B. von Parametern wie Temperatur, Lichteinwirkung, pH-Wert oder dem Aussetzen bestimmter Gase oder Schwermetallionen. Wichtige Wirkungen oder gewünschte Inhaltsstoffe sind nach Überschreiten einer zu definierenden Zeitdauer nicht mehr in ausreichender Menge verfügbar oder zerfallen sogar in schädliche Nebenprodukte.

Bei einem aus dem Stand der Technik bekannten Verfahren wird das Ausgangsprodukt Ascorbinsäure (Vitamin C) verwendet. Um Ascorbinsäure quantitativ nachzuweisen, gibt es zahlreiche kolorimetrische Methoden. Normalerweise verwendet man 2,4-Dinitrophenylhydrazin. Ascorbinsäure reagiert mit diesem zu einem Hydrazon, dessen Absorption gemessen werden kann. Darüber hinaus kann 2,2'-Bipyridin zum colorimetrischen Nachweis dienen. Hierbei macht man sich die Redukfionskraft der Ascorbinsäure zunutze, die Fe(III) zu Fe(II) reduziert. Fe(II) bildet dann mit 2,2*'*-Bipyridin einen farbigen Komplex Es sind auch einige fluorometrische Nachweismethoden bekannt.

Ascorbinsäure lässt sich auch durch Titration mit Tillmans' Reagenz (2,6-Dichlorphenolindophenol, abgekürzt DCPIP) nachweisen, bei der das Reagenz durch die Ascorbinsäure zu einer Leukoverbindung reduziert wird. Dabei ist ein Farbumschlag von tiefblau zu farblos zu beobachten. Diese Methode eignet sich für eine schnelle Bestimmung, die aber an die Genauigkeit oben genannter Wege nicht heranreicht. [Quelle: Wikipedial

Ascorbinsäure kann auch spezifisch mittels Oxidation durch das Enzym Ascorbinsäure-Oxidase nachgewiesen werden, wobei die Änderung der Lichtabsorption bei einer Wellenlänge von 245 nm gemessen wird.

Für Fisch, der kein Vitamin C enthält, muss ein anderes Ausgangsgangsprodukt als Frischemarker verwendet werden. Ein idealer Parameter für die Frischemessung in Salzwasserfischen ist Trimethylamin (TMA). TMA entsteht bei dem bakteriellen Abbau von Trimethyaminoxid (TMAO) und ist für den "fischigen" Geruch verantwortlich. TMAO kann aber auch enzymatisch in Dimethylamin (DMA) und Formaldehyd gespalten werden, wobei das Formaldehyd mit dem Gewebe reagiert und deswegen schlecht bestimmbar ist. Trimethyamin und Dimethlyamin werden normalerweise per HPLC bestimmt. Da jedoch diverse Fische verschiedene Ausgangskonzentrationen an TMAO aufweisen und sowohl der TMA als auch der DMA Stoffwechselweg eingeschlagen werden kann ist es notwendig eine genauere Methode zum Nachweis zu haben.

In Fleisch und Süßwasserfischen können verschiedene Ausgangsgangsprodukte als Frischemarker verwendet werden. Einer der einfachstem Frischemarker ist Histamin, das aus Histidin durch Decarboxylierung entsteht. Weitere biogene Amine, die als Frischemarker dienen können, sind beispielsweise aber nicht ausschließlich: Putrescin, Cadaverin und Tyramin. Putrescein entsteht durch Zersetzung von Ornithin, Cadaverin entsteht durch die Zersetzung von Lysin, Tyramin und durch die Zersetzung von Tyrosin. Da alle diese Frischemarker auf einen bakteriellen Zersetzungsprozess hinweisen, sind diese Stoffe gut geeignet die Lagerungstemperatur und die hygienischen Bedingungen bei der Verarbeitung von Fleisch und Fisch abzubilden. Auch in diesem Fall ist es sinnvoll den Gehalt der Ausgangssubstanz mit zu bestimmen, um so eine Information über den Grad der Zersetzung zu bekommen. In Histidin armem Gewebe ist es nicht verwunderlich, wenn der Gehalt an Histamin gering ist.

### Abbauprodukte von Nukleinsäuren

Der Abbau von Nukleinsäuren in Fleisch und Fisch ist ein guter Marker für die Frische, da dort die Nukleinsäuren über autolytische Prozesse und bakterielle Prozesse abgebaut werden. Stand der Technik ist hier der Nachweis über HPLC, der aber eine aufwendige Aufreinigung erfordert (Veciana-Nogues 1997, Determination of ATP related compounds *in fresh* and canned tuna *fish* by HPLC, Food Chemistry 59(3)). Andere Testsysteme erlauben nur die isolierte Bestimmung von individuellen Analyten (siehe Tabelle 1).

Der Abbau verläuft dabei von den energiereichen Triphopsphaten zu den energieärmeren Nukleotiden bzw den abgespaltenen Basen: ATP (Adenosintriphopshat) - ADP (Adenosindiphosphat) - AMP (Adenosinmonophosphat) - IMP (Inosinmonophosphat) - Ino (Inosin) - Hx (Hypoxanthin)

**Table 1: Frischetest unter Verwendung der Enzym-Technologie**

| Analyt(e) | Prinzip | Vorteil | Nachteil | Referenz |
|---|---|---|---|---|
| Hx | auf einem Test-streifen immobilisierte Enzyme (xanthine oxidase, X0) | schnell, außerhalb eines Labors einfach zu verwenden | halbquantitativ, nur geignet für Messung von Hx (Folge des Verderbens) | Jahns et al. (1976) |
| Hx, Ino | Teststreifen mit immobilisierten Enzymen | schnell, außerhalb eines Labors einfach zu verwenden | halbquantitativ, geringe Reproduzier-barkeit, begrenzt aufHx und Ino (Folge des Verderbens) | Ehira et al. (1986) |
| IMP, Ino, Hx | enzymbeschichtete Sauerstoffelektrode | schnell, genau | komplizierter und zeitaufwändiger als Teststreifen-Technologie | Karube et al. (1984) |
| K-index | Versuch mit gekoppelten Emzymen "KV-1 01 Freshness Meter" | schnell, Ergebnisse vergleichbar mit HPLC | es müssen Enzyme und Reagenzien gekauft werden, Kosten? | handel-sübliche Form, Orienta Electric, Niiza Saitama 352, Japan |
| K-index | enzymbeschichtete Sauerstoffelektrode "Microfresh" | schnell, Ergebnisse vergleichbar mit HPLC | Kosten? | handel-sübliche Form, Pegasus Instruments, Agincourt, ON, Canada |

Quelle: FAO FISHERIES TECHNICAL PAPER - 348 FOOD AND AGRICULTURE ORGANIZATION OF THE UNITED NATIONS, H. H. Huss Technological Laboratory, Ministry of Agriculture and Fisheries Denmark, FOOD AND AGRICULTURE ORGANIZATION OF THE UNITED NATIONS Rome, 1995

Im Stand der Technik werden einzelne Substanzen oder auch Gruppen verwandter Substanzen einzeln oder sequentiell gemessen. Jedoch muss für jede Substanz / Substanzgruppe eine eigene Analyse durchgeführt werden. Verwendete Verfahren dafür sind enzymatische Tests (ELISA), chromatographische Verfahren mit optischer Detektion (HPLC) oder photometrische Verfahren. Alle diese Verfahren haben den Nachteil, dass diese entweder nur einen Parameter erfassen können oder eine aufwendige Probenaufbereitung erfordern. Bei der HPLC Messung, bei der zwar für ein Ausgangsgangsprodukt mehrere ähnliche Zersetzungsprodukte gemessen werden, können, werden die Zersetzungsprodukte per Chromatographie getrennt und es muss im Vorfeld eine aufwendige Probenvorbereitung erfolgen. Gleichzeitig liegt das Problem vor, dass ein Frischeverlust aufverschiedenen Wegen stattfinden kann. So kann durch eine Wärmebelastung sowohl eine Autolyse als auch eine bakterielle Zersetzung stattfinden. Weitere Ursachen für einen Frischeverlust sind z.B. Oxidation und eine Zerstörung der Zellmembranen durch Einfrieren und wieder Auftauen.

In der vorliegenden Patentanmeldung wird unter einem Frischeverlust eine Minderung (Änderung) des Qualität eines Lebensmittel durch Alterungsvorgänge verstanden. Diese reduzieren z.B. den Nährwert, ändern den Geschmack und/oder Geruch, die optische und/oder haptischen Eigenschaften des Lebensmittels. Oftmals sind diese Vorgänge durch chemische / biochemische Reaktionen bedingt z.B. Wasserverlust, Hydrolyse, Abbau von Nährstoffen, stoffliche Umsetzung durch Mikroorganismen.

Aufgabe der Erfindung ist es, ein Verfahren der eingangs genannten Art zur Verfügung zu stellen, das es ermöglicht, einen Kennwert für die Frische und/oder den Frischeverlust eines Lebensmittels schnell und einfach, aber dennoch möglichst exakt zu bestimmen.

Erfindungsgemäß wird diese Aufgabe durch ein Verfahren zur Bestimmung eines Kennwerts für die Frische und/oder den Frischeverlust einer zumindest ein erstes Ausgangsgangsprodukt und ein zweites Ausgangsgangsprodukt enthaltenden Lebensmittel-Probe gelöst, bei dem beim Auftreten einer ersten Frischeverlustart das erste Ausgangsprodukt zersetzt und mindestens ein erster Metabolit gebildet wird, wobei beim Auftreten einer zweiten Frischeverlustart das zweite Ausgangsprodukt zersetzt und mindestens ein zweiter Metabolit gebildet wird, wobei für die Konzentration des ersten Ausgangsprodukts ein erster Ausgangsprodukt-Konzentrationswert und für die Konzentration des ersten Metaboliten ein erster Metabolit-Konzentrationswert gemessen wird, wobei für die Konzentration des zweiten Ausgangsprodukts ein zweiter Ausgangsprodukt-Konzentrationswert und für die Konzentration des zweiten Metaboliten ein zweiter Metabolit-Konzentrationswert gemessen wird, wobei die Ausgangsprodukt-Konzentrationswerte und die Metabolit-Konzentraflonswerte gleichzeitig gemessen werden, und wobei der Kennwert für die Frische der Lebensmittelprobe in Abhängigkeit vom Verhältnis des ersten Ausgangsprodukt-Konzentrationswerts zum ersten Metabolit-Konzentrationswert und vom Verhältnis des zweiten Ausgangsprodukt-Konzentrationswerts zum zweiten Metabolit-Konzentrationswert ermittelt wird.

Es werden also mehrere lebensmittelspezifischen Parameter, nämlich die Konzentrationen von mindestens zwei Ausgangsprodukten mit einer beschränkten Halbwertszeit und von mindestens zwei Metaboliten in einer vorzugsweise wasserhaltigen Lebenmittelprobe, gleichzeitig ermittelt, um so die Frische möglichst exakt zu bestimmen. Durch die gleichzeitige Erfassung der Konzentrationen, welche die verschiedenen Aspekte der Frische darstellen, wird der Aufwand der Messung möglichst gering gehalten. In vorteilhafter Weise werden außerdem die beim Stand der Technik auftretenden Probleme, wie z.B. aufwendige Probenvorbereitung bzw. multiple Bestimmungen, um die Analyte zu ermitteln, sowie eine Abnahme der Frische der Lebensmittelprobe zwischen zeitlich voneinander beabstandeten Konzentrationsmessungen, vermieden. Das Verfahren kann beispielsweise zum Zwecke des Verbraucherschutzes Anwendung finden, um auf der Verpackung von Lebensmitteln angebrachte Haltbarkeitsangaben zu überprüfen. Auch für die Qualitätskontrolle ist der Frischetest ein wichtiges Hilfsmittel.

Bei einer vorteilhaften Ausgestaltung des Verfahrens enthält die Lebensmittel-Probe mindestens ein weiteres Ausgangsgangsprodukt, das beim Auftreten einer weiteren Frischeverlustart unter Bildung eines weiteren Metabolits zersetzt wird, wobei für die Konzentration des weiteren Ausgangsprodukts ein weiterer Ausgangsprodukt-Konzentrationswert und für die Konzentration des weiteren Metaboliten ein weiterer Metabolit-Konzentrationswert gemessen wird, und wobei der Kennwert für die Frische der Lebensmittelprobe in Abhängigkeit vom Verhältnis des weiteren Ausgangsprodukt-Konzentrationswerts zum weiteren Metabolit-Konzentrationswert ermittelt wird. Dadurch kann der Kennwert für die Frische bzw. den Frischeverlust noch exakter ermittelt werden.

Bei einer Weiterbildung der Erfindung wird der Kennwert für die Frische in Abhängigkeit von den Frischeverlustarten ermittelt. In dem Kennwert sind also auch Informationen über die Art des Frischeverlusts enthalten, so dass anhand des Kennwerts die Ursache des Frischeverlusts ermittelt werden kann. Bevorzugt wird in dem Kennwert der Frischeverlust für die einzelnen Frischeverlustarten getrennt quantitativ erfasst, so dass beispielsweise ersichtlich ist, durch welche Frischeverlustart (wie z.B. Temperatureinwirkung bzw. Unterbrechung der Kühlkette, Einwirkung von Bakterien, Lichteinwirkung, Oxidation etc.) die Frische wie stark abgenommen hat.

Vorteilhaft ist, wenn die Ausgangsprodukt-Konzentrationswerte und die Metabolit-Konzentrationswerte mittels Fourier-Transformations-Infrarotspektroskopie (FTIR) gemessen werden. Die Messung kann dann im Zuge einer Routine-Qualitätsmessung von Facharbeitern durchgeführt werden und es wird kein spezielles Labor benötigt.

Bei einer bevorzugten Ausgestaltung der Erfindung wird gleichzeitig zur Messung der Ausgangsprodukt-Konzentrationswerte und zur Messung der Metabolit-Konzentrationswerte der Nährwert der Probe bestimmt. Da Frische bei Lebensmitteln aber neben einer Altersangabe auch ein Maß für den Nährwert und die gesundheitliche Unbedenklichkeit ist, ist diese Ausgestaltung des Verfahrens vorteilhaft. Bei Bedarf kann zusätzlich zum Nährwert auch der Vitamingehalt ermittelt werden.

Vorteilhaft ist, wenn zum Ermitteln der Metabolit-Konzentrationswerte und der Ausgangsprodukt-Konzentrationswerte Messwerte erfasst werden, wenn die Messwerte matrixspezifisch korrigiert werden, und wenn die Konzentrationswerte mit Hilfe der korrigierten Messwerte ermittelt werden. Dabei wird davon ausgegangen, dass sich in einem Lebensmittel (der Matrix) die spezifischen Infrarot-Absorptionsbanden einer Substanz durch die Interaktion mit den anderen Bestandteilen des Lebensmittels verändern. Ändern sie diese (z.B. wenn Milch sauer wird) dann können diese Interaktionen das IR-Spektrum der zu analysierenden Substanz ändern. Diese Effekte werden als Matrixeffekte bezeichnet. Bei der matrixspezifisch Korrektur werden diese Änderungen mit entsprechenden mathematischen Verfahren kompensiert, z.B. durch Verwendung eines Look Up Table für die pH-Wert bedingten Veränderungen. Durch die matrixspezifische Korrektur kann Hintergrundrauschen kompensiert werden. Somit kann der Kennwert für die Frische und/oder den Frischeverlust noch genauer ermittelt werden. Bevorzugterweise wird eine Vielzahl von Parametern für mehrere verschiede Arten der Alterung (thermisch, bakteriell, autolytisch, chemisch) erfasst und ausgewertet. Bei der Messung solcher Parameter mittels FTIR besteht das Problem, dass Wasser in einem großen Bereich des Infrarot-Spektrums eine starke Absorbtion aufweist. Dadurch ist es extrem schwierig von gering konzentrierten Substanzen ein Spektrum in komplexen Matrices wie z.B. Lebensmitteln aufzunehmen. Es zeigte sich in vielen Messungen, dass durch die Alterung der Lebensmittelprobe die Messergebnisse verändert werden und insbesondere die spektrale Signatur definierter Inhaltsstoffe verändert wird. Dadurch entsteht die Notwendigkeit einer altersgemäßen Korrektur der Proben. Werte, die dabei eine Rolle spielen sind z.B. freie Säuren und Basen, die an Analyte binden können und so deren spektrale Eigenschaften verändern. Überraschenderweise zeigte es sich, dass sich die modifizierten Spektren in diesen Lebensmitteln mathematisch so kompensieren lassen, dass eine Quantifizierung weiterhin möglich ist, wobei die absolute Größe der Änderung des Spektrums ebenfalls als Maß für die Veränderung (= Frischeverlust) verwendet werden kann. Insbesondere lässt sich durch Messung in von extrem dünnschichtigen Flüssigkeitsfilmen (2-50 µm, insbesondere 10-30 µm) und die alterungsgemäße Korrektur der Spektren die Nachweisgrenze für quantiatative Infrarot-Spektroskopie auf unter 50 µM reduzieren. Die Restmenge des Stoffes und die Menge der Zerfallsprodukte sind ein Maß für die Ausgangskonzentration in dem Lebensmittel. Das Verhältnis Restmenge zu Ausgangsmenge ergibt den Kennwert für die Frische des Lebensmittels. Die Art der Zerfallsstoffe erlaubt einen Rückschluss auf die Weise in der das Lebensmittel gealtert ist, z.B. Hitzeeinwirkung, Oxidation, bakterielle Zersetzung.

Das erfindungsgemäße Verfahren ermittelt die Frische von Lebensmitteln anhand von Spektren der Flüssigkeiten der Nahrungsmittel, z.B. von Saft, indem ein spezifischer Inhaltstoff im Verhältnis zu seinen Verfallsprodukten betrachtet wird. Dies geschieht erfindungsgemäß bevorzugt mit IR Spektroskopie, ist aber ebenso mit anderen spektroskopischen oder auch chromatographischen Verfahren möglich. Das erfindungsgemäße Verfahren funktioniert bei allen Stoffen oder Ausgangsgangsprodukten, die eine Verfallssensitivität aufweisen, insbesondere durch Wärme, Licht, pH-Wert, Enzyme, Mikroorganismen oder insbesondere katalytisch wirksame Substanzen, und die ein oder mehrere stabile Verfallsprodukte oder Metaboliten haben. Da die Metabolite je nach Verfallsursache variieren, können auch Aussagen getroffen werden, in welcher Weise das Produkt beansprucht worden ist, z.B. zu viel Wärme oder Enzyme oder Ähnliches.

In einer bevorzugten Ausführungsform der Erfindung wird die Bestimmung des Vitamin C Gehaltes und der Zerfallsprodukte mit Hilfe der Infrarotspektroskopie durchgeführt. Vitamin C (Ascorbinsäure) wird reversibel zu Dehydroascorbinsäure (DHA) oxidiert und irreversibel zu Diketogulonsäure (DKG) und Oxalsäure zersetzt. Unter Hitze wird auch Hydoxymethylfurfural (HMF) gebildet, was einen braunen Farbstoff bildet. Es wird eine flüssige Lebensmittelprobe in einer Microflusszelle (Dicke ca 2 µm bis 50 µm, bevorzugt 5-15 µm) im mittleren Infrarotbereich vermessen und die Zerfallsprodukte und das Vitamin C werden aufgrund Ihrer spezifischen Spektren identifiziert. Solche Flusszellen sind z.B. von der Firma Bruker kommerziell erhältlich. Dazu ist vor allem die "Fingerprint" Region (ca. 2 µm - 40 µm) der Infrarotabsorption nützlich. Auf diese Weise kann mit einer Messung und der anschließenden spektralen Analyse die Menge der relevanten Zerfallsprodukte schnell gemessen werden. Insbesondere wird dabei der Gehalt der Probe an Vitamin C, DHA, DKG und HMF gemessen. Die Summe aus Vitamin C und DHA gibt dabei den effektiven Gehalt an Vitamin C für die Ernährung an, wohingegen das Verhältnis vom Vitamin C zur Summe der Zerfallsprodukte ein inverses Maß für die Frische darstellt. In einer bevorzugten Ausführungsform werden die gleichzeitig mit gemessenen bakteriellen Zersetzungsprodukte Milchsäure und Ethanol mit analysiert und mit Ihren Ausgangsprodukten Glucose und Maleinsäure in Relation gesetzt.

In einer weiteren Ausführungsform der Erfindung werden andere Zerfallsprozesse in den Lebensmitteln analysiert, wobei zur Bestimmung der Frische immer ein Verhältnis Metabolit (Edukt) in Relation zum Ausgangsgangsprodukt gebildet wird. Als Beispiel sei hier HMF in Honig genannt. Eine geringe Menge an HMF im Honig ist ein Indikator für dessen Frische und Naturbelassenheit. Ein hoher HMF-Wert weist auf länger anhaltende Erwärmung oder Lagerung hin. Wenn Honig erhitzt wird, bildet sich aus Fruchtzucker HMF. Der HMF-Gehatt in frisch geschleudertem Honig ist sehr gering und steigt bei korrekter Lagerung, je nach pH-Wert und Lagertemperatur um ca. 2-3 mg/kg pro Jahr an. Lagerung bei Zimmertemperatur (21 °C) kann den HMF-Gehalt in einem Jahr bereits auf 20 mg/kg erhöhen. Die EU hat einen HMF-Grenzwert von maximal 40 mg/kg für Honig, der unter europäischen Bedingungen produziert wurde, festgelegt. Einige nationale Imkerverbände fordern sogar noch niedrigere Werte, z. B. erlaubt der Deutsche Imkerbund höchstens 15 mg/kg für sein Gütesiegel "Echter Deutscher Honig".

Im Stand der Technik wird HMF im Honig wird meistens mit HPLC- oder dem sogenannten Winkler-Verfahren nachgewiesen. Seit 2009 ist ein Schnelltest von der Merck KGaA zur Bestimmung von HMF erhältlich. Bei dem "Reflectoquant® HMF" genannten Test wird eine geringe Menge Honig im Verhältnis 1:4 mit destilliertem Wasser verdünnt, ein Teststreifen in die Probe getaucht und dann in einem RQftex-Reflektometer gemessen. Alle diese Tests haben die Einschränkung, dass immer nur eine Art von Parameter, bzw eine Art von Alterung gemessen wird.

Das erfindungsgemäße Verfahren ist nicht auf bestimmte Stoffe beschränkt, sondern es eignen sich alle Substanzen, die unter bestimmten Bedingungen zu definierten Zerfallsprodukten umgewandelt werden. Solche Substanzen sind z.B. (aber nicht ausschließlich): Thiamin, Pyrodoxin, Cyancobolamin, Phenole, Indol-Essigsäure (Abbau zu 2-Acetaminophenon), Apfelsäure (Abbau der Apfelsäure durch malolaktische Gärung zu Essigsäure), Mannit (Enzymatische Umwandlung von Fructose zu Mannit), oxidierbare Fettsäuren ("Ranzigkeit").

Das Verfahren ist besonders geeignet für flüssige Lebensmittel wie z.B. Wein, Bier, Saft, aber auch für andere Lebensmittel, sofern die zu untersuchenden Inhaltsstoffe daraus extrahiert werden können. Dies kann z.B. durch homogenisieren von Fleisch mit einer Pufferlösung oder auch durch Extraktion mit organischen Lösungsmitteln geschehen. Da in dem Verfahren ein Verhältnis aus Ausgangsgangsprodukt zu Metabolit gebildet wird ist vor allem bei Extrakten die Effizienz nicht von ausschlaggebender Wichtigkeit, da durch die Verrechnung von Ausgangsgangsprodukt zu Metabolit(en) eine dimensionslose Einheit entsteht. Ist das Lebensmittel fest oder schwer extrahierbar, so kann in einer weiteren bevorzugten Ausführungsform der Erfindung die Bestimmung der Paramter mittels ATR-IR Spektroskopie durchgeführt werden. Dazu wird z.B. ein ATR-Kristall auf die Probe gedrückt um dann im Bereich der evaneszenten Wellen die Absorption im IR (MIR) Bereich zu messen. Entsprechende ATR Sonden gibt es kommerziell zu kaufen.

In einer besonders bevorzugten Ausführungsform ist nicht nur das Spektrum der Reinsubstanzen bekannt und auch das Spektrum der gesuchten Substanzen in der relevanten Matrix (Vitamin C in Apfelsaft), sondern auch das Spektrum des entsprechend gealterten Lebensmittels. Die alterungstypischen spektralen Änderungen können dann z.B. mittels einer sog. Hauptkomponentenanalyse (PCA) ermittelt werden und als zusätzliches oder auch alleiniges Identifikationskriterium für das Alter der Lebensmittelprobe dienen. In diesem Fall sind Faktoren wie die einzelnen Substanzen (z.B. Vitamin C, DHA..) in den jeweiligen Spektren mit abgebildet.

In einer weiteren bevorzugten Ausführungsform werden zudem die Faktoren, die den Nährwert des Lebensmittels ausmachen in der gleichen Messung mitbestimmt. Dies ist möglich, da diese Substanzen alle einen Fingerabdruck im IR-Bereich haben (Signatur), und so mit der gleichen Messung (d.h. gleichzeitig) mit erfasst werden können. Die erfassten Werte müssen dann noch matrixspezifisch ausgewertet werden, da die Matrix (d.h. das Lebensmittel) einen deutlichen Einfluss auf die IR-Spezifische Signatur der Einzelkomponenten hat. Faktoren, die den Nährwert ausmachen, sind unter anderem Makronährstoffe und Mikronährstoffe. Diese Substanzen können mittles FT-IR exakt bestimmt werden.

Die Erfindung betrifft also ein Verfahren zur Bestimmung der Frische einer Probe, bei dem
- die Konzentration eines ersten Metaboliten der ein Marker für eine Art des Frischeverlustes ist bestimmt wird,
- die Konzentration mindestens eines weiteren Metaboliten der ein Marker für eine weiter Art des Frischeverlustes ist bestimmt wird,
- die Konzentration der Ausgangsgangsprodukte für gemessenen Metaboliten bestimmt wird, und
- Bewertung des Frischeverlustes aus dem Verhältnis der Metabolite und deren Ausgangsprodukt sowie der metabolitspezifischen Art des Frischeverlustes.

Die Bestimmung der Metaboliten und deren Ausgangsstoffe kann gleichzeitig stattfinden, vorzugsweise mittels FT-IR.

Nachfolgend wird die Erfindung anhand eines Ausführungsbeispiels näher beschrieben.

Bei einem ersten Ausführungsbeispiel wird von einem zu prüfenden Apfelsaft eine Probe von 10 ml zu Analyse entnommen. Davon werden 1 ml durch einen Filter der Porengröße 0,4 µm filtriert. Anschließend werden davon ca. 150 µl in eine FTIR Mikro-Flusszelle injiziert und mittels FTIR vermessen mittels Licht der Wellenlänge 1-11 µm. Aus dem komplexen Spektrum werden dann mit Hilfe einer auf einem Computer laufenden Analysesoftware die Gehalte an Vitamin C und den potentielle Zerfallsprodukten von Vitamin C analysiert, sowie auf andere Hilfsmarker wie z.B. HMF.
1. Bestimmen des Gehalts an Vitamin C unter Verwendung von matrixspezifischen Korrekturen.
2. Bestimmen des Gehalts an Verfallsprodukten von Vitamin C unter Verwendung von matrixspezifischen Korrekturen.
3. Bilden der aus Vitamin C und Verfallsprodukten von Vitamin C resultierenden Gesamtkonzentration an ursprünglichem Vitamin C in Lebensmitteln.
4. Berechnen des Frischegrades des Lebensmittels aus Verhältnis von derzeitigem und ursprünglichem Gehalt von Vitamin C.
5. Bewerten der Verfallsprodukte in
   a) Thermischen Verfall
   b) Oxidativen Verfall
   c) Enzymatische Degradation (inklusive bakterieller Abbau)
   d) Reversible Zerfallsprodukte (können im Organismus wieder in Vitamin C zurückverwandelt werden)

In diesem Beispiel zeigte es sich, dass ein Apfelsaft einen HMF Gehalt vom 30 ml/l aufweist, einen DHA Gehalt von 12 mg/l und einen Vitamin C Gehalt von 45 mg/l. Die anderen Werte sind unauffällig. Somit wird ein Ausgangsgehalt von 87 mg/l Vitamin C ermittelt. Der Verlust an Vitamin C und der Hohe HMF Gehalt zeigen, dass der Apfelsaft erhitzt wurde. Als Kennwert für die Frische wird also ermittelt, dass der Apfelsaft nicht mehr zum Verzehr geeignet ist. Da kaum oxidativer Zerfall vorliegt, wurde der Apfelsaft wahrscheinlich nach dem Verpacken in eine Verpackung der Wärme ausgesetzt.

Als zusätzliche Option werden aus der Messung auch die Gehalte an Milchsäure und Maleinsäure sowie Glucose und Ethanol identifiziert und ausgewertet. Liegen Verhältnisse von Maleinsäure (Maleat)/ Milchsäure (Lactat) unter 20, so hat in dem Apfelsaft eine maleolaktische Gärung stattgefunden. In diesem Fall wird in dem Kennwert für die Frische gespeichert, dass der Apfelsaft durch bakterielle Einwirkung gealtert ist.

Bei einem zweiten Ausführungsbeispiel wird die Frische von Fisch bestimmt. Die erfindungsgemäße Lösung besteht hier in der Gewinnung eines flüssigen Extraktes, der mit Phosphatpuffer auf pH 7 gepuffert wird und dann mittels MIR-FTIR aufTMAO, TMA und DMA untersucht wird. Das Verhältnis TMAO zu TMA und DMA ist ein weit besseres Frischemaß als der absolute Gehalt an TMA oder DMA wie bisher untersucht.

Dazu wird eine repräsentative Menge des Fisches (Nordsee Wittling) entnommen (5g) und mit PBS Puffer pH 7 homogenisiert (5 ml). Das Homogenisat wird durch Spritzenvorsatzfilter filtriert und dann davon 200 µl in eine AquaSpec Flusszelle zur Messung im mittleren Infrarotbereich injiziert (FTIR Messung). Dabei werden alle nachweisbaren Inhaltsstoffe des Extraktes mit einer Nachweisgrenze von ca. 1 µM - 10 µM mittels einer Messung in ca. zwei Minuten bestimmt. Die gewünschten Faktoren werden dann von der bereits erwähnten Software aufbereitet und an den Benutzer weitergegeben. Bei der Messung zeigte sich ein TMA Wert von 3,7 mM und ein TMAO Wert vom 7,4 mM. Damit ist der Zerfall eines signifikanten Anteils des TMAO nachgewiesen. Als Kennwert für die Frische wird also ermittelt, dass der Fisch nicht mehr zum Verzehr geeignet ist.

Als zusätzliche Option wird der Gehalt an Histamin und Histidin aus der gleichen Messung bestimmt und ausgewertet. Ist der Grenzwert von 200 mg/kg Histamin überschritten, so ist die probe nach EU Lebensmittelrecht nicht mehr zum Verzehr geeignet und muss wegen starker bakterieller Zersetzung aus dem Verkehr genommen werden. Ist der Histamingehalt geringer, so wird ein Score Histidin /Histamin gebildet. Je geringer dieser Score ist, desto weniger mit Balkterien ist der Fisch belastet.

## Patentansprüche

1. Verfahren zur Bestimmung eines Kennwerts für die Frische und/oder den Frischeverlust einer zumindest ein erstes Ausgangsgangsprodukt und ein zweites Ausgangsgangsprodukt enthaltenden Lebensmittel-Probe, wobei beim Auftreten einer ersten Frischeverlustart das erste Ausgangsprodukt zersetzt und mindestens ein erster Metabolit gebildet wird, wobei beim Auftreten einer zweiten Frischeverlustart das zweite Ausgangsprodukt zersetzt und mindestens ein zweiter Metabolit gebildet wird, wobei für die Konzentration des ersten Ausgangsprodukts ein erster Ausgangsprodukt-Konzentrationswert und für die Konzentration des ersten Metaboliten ein erster Metabolit-Konzentrationswert gemessen wird, wobei für die Konzentration des zweiten Ausgangsprodukts ein zweiter Ausgangsprodukt-Konzentrationswert und für die Konzentration des zweiten Metaboliten ein zweiter Metabolit-Konzentrationswert gemessen wird, wobei die Ausgangsprodukt-Konzentrationswerte und die Metabolit-Konzentrationswerte gleichzeitig gemessen werden, und wobei der Kennwert für die Frische der Lebensmittelprobe in Abhängigkeit vom Verhältnis des ersten Ausgangsprodukt-Konzentrationswerts zum ersten Metabolit-Konzentrationswert und vom Verhältnis des zweiten Ausgangsprodukt-Konzentrationswerts zum zweiten Metabolit-Konzentrationswert ermittelt wird.

2. Verfahren nach Anspruch 1, wobei die Lebensmittel-Probe mindestens ein weiteres Ausgangsgangsprodukt enthält, das beim Auftreten einer weiteren Frischeverlustart unter Bildung eines weiteren Metabolits zersetzt wird, wobei für die Konzentration des weiteren Ausgangsprodukts ein weiterer Ausgangsprodukt-Konzentrationswert und für die Konzentration des weiteren Metaboliten ein weiterer Metabolit-Konzentrationswert gemessen wird, und wobei der Kennwert für die Frische der Lebensmittelprobe in Abhängigkeit vom Verhältnis des weiteren Ausgangsprodukt-Konzentrationswerts zum weiteren Metabolit-Konzentrationswert ermittelt wird.

3. Verfahren nach Anspruch 1 oder 2, wobei der Kennwert für die Frische in Abhängigkeit von den Frischeverlustarten ermittelt wird.

4. Verfahren nach einem der Ansprüche 1 bis 3, wobei die Ausgangsprodukt-Konzentrationswerte und die Metabolit-Konzentrationswerte mittels Fourier-Transformations-Infrarotspektroskopie gemessen werden.

5. Verfahren nach Anspruch 4, wobei zum Ermitteln der Metabolit-Konzentrationswerte und der Ausgangsprodukt-Konzentrationswerte Messwerte erfasst werden, wobei die Messwerte matrixspezifisch korrigiert werden, und wobei die Konzentrationswerte mit Hilfe der korrigierten Messwerte ermittelt werden.

6. Verfahren nach einem der Ansprüche 1 bis 5, wobei eine Art des Frischeverlustes die Autolyse ist.

7. Verfahren nach Anspruch 1 bis 6, wobei gleichzeitig zur Messung der Ausgangsprodukt-Konzentrationswerte und zur Messung der Metabolit-Konzentrationswerte der Nährwert der Probe bestimmt wird.

8. Verfahren nach einem der Ansprüche 1 bis 7, wobei das erste Ausgangsgangsprodukt Ascorbinsäure, ein erster erster Metabolit Dehydroascorbinsäure, ein zweiter erster Metabolit Diketogulonsäure und ein dritter erster Metabolit Hydoxymethylfurfural ist, wobei das zweite Ausgangsgangsprodukt Milchsäure und der zweite Metabolit Glucose oder wobei das zweite Ausgangsgangsprodukt Ethanol und der zweite Metabolit Maleinsäure ist.
